(19) Europäisches Patentamt
European Patent Office
Office européen des brevets

(11) Veröffentlichungsnummer: **0 215 257**
**A1**

(12) **EUROPÄISCHE PATENTANMELDUNG**

(21) Anmeldenummer: **86110673.0**

(22) Anmeldetag: **01.08.86**

(51) Int. Cl.⁴: **C 08 G 65/44**, C 07 C 43/295

(30) Priorität: **14.08.85 DE 3529093**

(43) Veröffentlichungstag der Anmeldung: **25.03.87 Patentblatt 87/13**

(84) Benannte Vertragsstaaten: **DE FR GB IT**

(71) Anmelder: **BAYER AG, Konzernverwaltung RP Patentabteilung, D-5090 Leverkusen 1 Bayerwerk (DE)**

(72) Erfinder: **Tacke, Peter, Dr., Brandenburger Strasse 12, D-4150 Krefeld (DE)**
Erfinder: **Freitag, Dieter, Dr., Hasenheide 10, D-4150 Krefeld (DE)**

(54) **Verfahren zur Herstellung von mono- und bifunktionellen Oligophenylenoxiden.**

(57) Die Erfindung betrifft ein Verfahren zur Herstellung von Oligophenylenoxiden durch katalytische Oxidation von Phenolen mit sauerstoffhaltigen Gasen in Alkoholen.

EP 0 215 257 A1

0215257



BAYER AKTIENGESELLSCHAFT 5090 Leverkusen, Bayerwerk
Konzernverwaltung RP
Patentabteilung Eck/Th-c

Verfahren zur Herstellung von mono- und bifunktionellen Oligophenylenoxiden

Die Erfindung betrifft ein Verfahren zur Herstellung von Oligophenylenoxiden durch katalytische Oxidation von Phenolen mit sauerstoffhaltigen Gasen in Alkoholen

Mono- und bifunktionelle Oligophenylenoxide sind bekannt. Sie können beispielsweise durch Oxidation von alkylsubstituierten Phenolen mit Sauerstoff bzw. Sauerstoff-haltigen Gasen in Gegenwart spezieller Katalysatorsysteme hergestellt werden. Die Verfahren sind beispielsweise in den US-PS 31 40 675 und 42 34 706 und der DE-OS 33 08 421 beschrieben. Danach dienen halogenierte Aliphaten und Aromaten als Lösungsmittel bei diesen Oxidationen.

Aus der DE-OS 2 126 434 ist bekannt, Polyphenylenoxide durch katalytische Oxidation in Alkoholen herzustellen.

Die genannten Lösungsmittel zeigen den Nachteil, daß in ihnen sowohl das Monomer als auch die Oligo- und Polymeren leicht löslich sind. Hieraus ergibt sich, daß im Verlauf der Oxidation Oligophenylenoxide auch dann noch der Einwirkung des Sauerstoffes ausgesetzt sind, wenn sie längst das gewünschte Molgewicht erreicht haben.

Es wurde nun gefunden, daß Oligophenylenoxide mit engen Molgewichtsverteilungen ohne Überoxidation hergestellt werden können, wenn aliphatische $C_1$-$C_5$-Alkohole als Lösungsmittel verwendet werden. Durch Zusatz von Wasser kann die Löslichkeit der Oligophenylenoxide in den Alkoholen gesteuert werden, wodurch sich bestimmte Molgewichte gezielt herstellen lassen. Das ausgefallene Material kann z.B. durch Absaugen leicht abgetrennt und durch Waschen gereinigt werden.

Gegenstand der Erfindung ist ein Verfahren zur Herstellung von mono- und bifunktionellen Oligophenylenoxiden mit einem mittleren Molgewicht $\bar{M}_n$ <2800 der Formel (I)

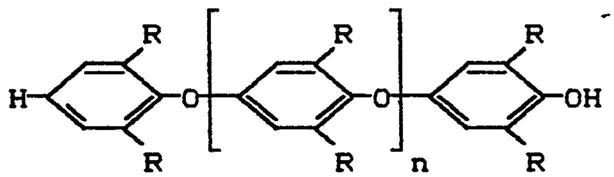

(I),

in welcher die Reste R unabhängig voneinander für Wasserstoff, $C_1$-$C_5$-Alkyl stehen und n für die Zahl 0, 1, 2, 3, 4 oder 5 steht

und Bisphenolen der Formel (II) mit einem mittleren Molgewicht $\bar{M}_n$ <2800

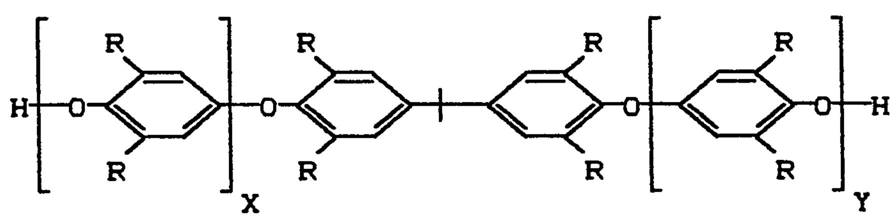

(II),

in welcher

R die bei Formel (I) angegebene Bedeutung hat,

X für eine ganze Zahl von 0 bis 30 und

Y für eine ganze Zahl von 0 bis 30 steht,

mit der Maßgabe, daß entweder X oder Y verschieden von der Zahl 0 sein muß,

durch katalytische Oxidation von Phenolen mit Sauerstoffhaltigen Gasen, dadurch gekennzeichnet, daß als Lösungsmittel $C_1$-$C_5$-aliphatische monofunktionelle Alkohole, gegebenenfalls unter Zusatz von 1 bis 30 Vol-% Wasser verwendet werden.

Vorzugsweise steht X und Y in Formel (II) unabhängig voneinander für die Zahlen 1, 2, 3, 4, 5, 6, 7, 8, 9, 10, 11, 12, 13, 14, 15.

Die erzielten Molgewichte ($\bar{M}_n$) der hergestellten Oligomeren liegen im Bereich von 300 bis 2.800, vorzugsweise 500 bis 2.000.

Die im erfindungsgemäßen Verfahren einsetzbaren Phenole sind z.B. aus den US-PS 31 40 675 und 42 34 706 und der DE-OS 33 08 421 bekannt. Bevorzugt sind Phenole mit 1 bis 2 Alkyl-Substituenten mit 1 - 5 C-Atomen, vorzugsweise in o-Position zur phenolischen OH-Gruppe, z.B. 2,6-Dimethylphenol.

Zur Herstellung von bifunktionellen Oligophenylenoxiden sollte mindestens ein Diphenol mitverwendet werden, z.B. 3,5,3',5'-Tetramethyl-Bisphenol-A (BPA).

Als Katalysatoren können im erfindungsgemäßen Verfahren übliche, bekannte Katalysatoren, vorzugsweise Kupfer- und/oder Manganhalogenide wie $CuCl_2$, CuCl, $MnCl_2$ verwendet werden und übliche bekannte Cokatalysatoren, z.B. Amine wie Triethylamin, N-Methylpiperidin verwendet werden. Erfindungsgemäß verwendbare Katalysatoren und Cokatalysatoren sind z.B. in der DE-A-33 08 421 beschrieben.

Als Lösungsmittel können aliphatische Alkohole mit 1 bis 15 C-Atomen, wie Methanol, Ethanol, n-Propanol, i-Propanol, n-Butanol, i- Butanol, tert.-Butanol, Pentanol, Neopentylalkohol, bevorzugt mit 1 bis 4C-Atomen, verwendet werden. .

Im erfindungsgemäßen Verfahren können Phenole der allgemeinen Formel (III)

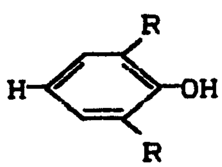

(III),

in welcher R die bei Formel (I) angegebenen Bedeutung hat,

und (IV)

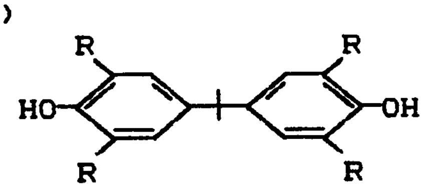

(IV),

in welcher R die bei Formel (I) angegebenen Bedeutung hat,

eingesetzt werden.

Das erfindungsgemäße Verfahren kann im Temperaturbereich von -80 bis 100°C, gegebenenfalls bei erhöhtem Druck bis 10 bar, ausgeführt werden. Bevorzugt sind Temperaturen im Bereich von 0 bis +40°C und Normaldruck.

Zur Durchführung des erfindungsgemäßen Verfahrens werden der Katalysator und der Cokatalysator in dem gewählten Alkohol, gegebenenfalls unter Mitverwendung von Wasser, vorgelegt und mit einem Sauerstoff-haltigem Gas, vorzugsweise Luft, oxidiert. Dann wird das Phenol gegebenenfalls mit Bisphenol versetzt und weiteroxidiert bis die Oligophenylenoxide ausgefallen sind. Das Produkt wird abgesaugt und gewaschen. Die Ausbeute beträgt bis zu 95 %. Gegebenenfalls kann die getrennte Oxidation des Katalysatorsystems entfallen.

Zur Entfernung des Katalysators aus dem Produkt kann gegebenenfalls nochmals gelöst und wieder ausgefällt werden. Nach Waschen der Lösung kann dann eingedampft werden.

Bei Verwendung von Phenolen der Formel (III) entstehen vorzugsweise monofunktionelle Polyphenylenoxide der Formel (I).

Werden im erfindungsgemäßen Verfahren Phenole der Formel (III) und der Formel (IV) eingesetzt, entstehen vorzugsweise bifunktionelle Polyphenylenoxide der Formel (II).

In diesem Falle werden vorzugsweise pro Mol Phenol der Formel (IV) etwa zwei Mol Phenol der Formel (III) eingesetzt.

Die nach dem erfindungsgemäßen Verfahren hergestellten Oligophenylenoxide können z.B. als Brandschutzmittel für verschiedene andere Werkstoffe (z.B. Polyamid) eingesetzt oder als Comonomere in Cokondensate eingebaut werden. Cokondensate der genannten Art sind durch besonders gute Wärmeformbeständigkeit gekennzeichnet.

Beispiel 1

1,5 g CuCl und 2 g N,N-Dimethyl-4-aminopyridin wurden mit 120 ml 98 Vol.-%igem Ethanol (2 % Wasser) verrührt und 30 min lang wurde Luft (50 ml/h) hindurchgeleitet.

Nach Zugabe von 122 g 2,6-Dimethylphenol und weiteren 600 ml Ethanol wurde weiter Luft hindurchgeleitet. Nach ca. 5 Std. begann eine hellbraune Substanz auszufallen. Nach weiteren 7 Std. hatte sich ein dicker Brei gebildet.

Die Substanz wurde abgesaugt, mit 50 ml Ethanol gewaschen, dann 12 Std. unter $N_2$ mit 300 ml 2 %iger wäßriger HCl verrührt, wieder abgesaugt und mit Wasser gründlich gewaschen und im Vakkumtrockenschrank bei 70°C getrocknet. Die Ausbeute betrug 107,5 g hellgelbes Pulver.

Die osmometrische Molekulargewichtsbestimmung des monofunktionellen Oligophenylenoxides ergab $\bar{M}_n$ 1813.

Beispiel 2

Die Umsetzung von Beispiel 1 wurde wiederholt, jedoch 92 Vol.-%iges Ethanol verwendet. Die Ausbeute betrug 105,8 g hellgelbes Pulver mit $\bar{M}_n$ 1521.

Beispiel 3

Die Umsetzung von Beispiel 1 wurde mit 85 %igem Ethanol wiederholt. Die Ausbeute lag bei 108,2 g vom $\bar{M}_n$ 1287.

Beispiel 4

Die Umsetzung von Beispiel 1 wurde mit 97,6 %igem Methanol wiederholt. Es wurden 106,3 g Pulver vom $\bar{M}_n$ 1392 erhalten.

Beispiel 5

Bei Verwendung von Isopropanol (98,6 Vol.-%ig) lag die Ausbeute bei 103,7 g vom $\bar{M}_n$ 1878.

Beispiel 6

Zur Herstellung von bifunktionellem Oligphenylenoxid wurden 1,5 g CuCl und 2 g N,N-Dimethyl-4-aminopyridin mit 120 ml 90 %igem Ethanol (Vol-%) verrührt und 30 min lang Luft (50 l/h) hindurchgeleitet.

Anschließend wurden 28,5 g 2,2-Bis-(3,5-dimethyl-4-hydroxyphenyl)-propan, 97,7 g 2,6-Dimethylphenol und 600 ml 90 %igem Ethanol zugesetzt und die Zuleitung von Luft fortgesetzt. Nach 13,5 Std. war ein dicker Brei entstanden.

Die Aufarbeitung erfolgte wie in Beispiel 1 angegeben. Das bifunktionelle Oligophenylenoxid stellte ein hellgelbes Pulver dar.
Die Ausbeute betrug 106,4 g von $\bar{M}_n$ 1248 (osmometrisch in $CH_2Cl_2$ bestimmt).
Eine gelchromatografische Analyse zeigte nur 1,3 Gew.-% monofunktionelle Anteile.

## Patentansprüche

1) Verfahren zur Herstellung von mono- und bifunktionellen Oligophenylenoxiden mit einem mittleren Molgewicht $\bar{M}_n$ <2800 der Formel (I)

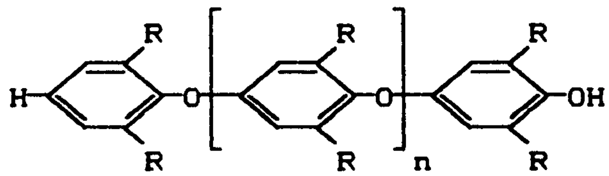

(I),

in welcher die Reste R unabhängig voneinander für Wasserstoff, $C_1$-$C_5$-Alkyl stehen und n für eine ganze Zahl von 0 bis 5 steht

und Bisphenolen der Formel (II) mit einem mittleren Molgewicht $\bar{M}_n$ <2800

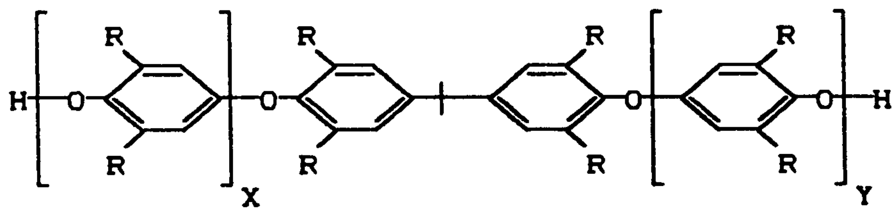

(II),

in welcher

R die bei Formel (I) angegebene Bedeutung hat,

X für eine ganze Zahl von 0 bis 30 und

Y für eine ganze Zahl von 0 bis 30 steht,

mit der Maßgabe, daß entweder X oder Y verschieden von der Zahl 0 sein muß, durch katalytische Oxidation von Phenolen mit Sauerstoff-haltigen Gasen, dadurch gekennzeichnet, daß als Lösungsmittel $C_1$-$C_5$-aliphatische monofunktionelle Alkohole, gegebenenfalls unter Zusatz von 1 bis zu 30 Vol-% Wasser verwendet werden.

2) Verfahren nach Anspruch 1, dadurch gekennzeichnet, daß Phenole der Formel (III)

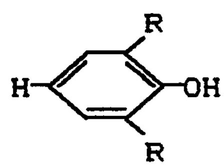
(III)

in welcher

R unabhängig voneinander für Wasserstoff und $C_1$-$C_5$-Alkyl stehen,

eingesetzt werden.

3) Verfahren nach Anspruch 1, dadurch gekennzeichnet, daß Bisphenole der Formel (IV)

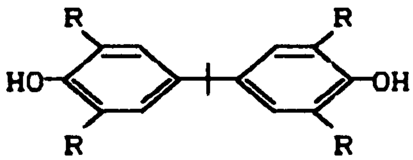

(IV)

in welcher

R unabhängig voneinander für Wasserstoff und $C_1$-$C_5$-Alkyl stehen,

eingesetzt werden.

4) Verfahren nach Anspruch 1, dadurch gekennzeichnet, daß pro Mol der Formel (III) etwa 2 Mol Phenol der Formel (IV) eingesetzt werden.

5) Verfahren nach Anspruch 1, dadurch gekennzeichnet, daß Luft als Sauerstoff-haltiges Gas verwendet wird.

Europäisches Patentamt

**EUROPÄISCHER RECHERCHENBERICHT**

0215257

Nummer der Anmeldung

EP 86 11 0673

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 122 412 (BAYER AG) * Anspruch 1; Beispiele * --- | 1-3 | C 08 G 65/44<br>C 07 C 43/295 |
| A | DE-B-1 745 432 (SUMITOMO CHEMICAL CO. LTD.) * Ansprüche 1, 4, 6; Spalte 4, Zeilen 37-39; Spalte 5, Zeilen 18-25, 33-35, 56-60 * --- | 1,2,5 | |
| D,A | DE-A-2 126 434 (ASAHI-DOW LTD.) * Beispiele 7, 11 * --- | 1,2 | |
| A | US-A-4 059 568 (G.D. COOPER) * Ansprüche 1-4, 7-9; Beispiele 1, 4; Tabelle 1 * --- | 1,2 | |
| A | US-A-3 804 864 (T.F. RUTLEDGE) * Anspruch 1; Beispiele 1-6 * --- | 1 | **RECHERCHIERTE SACHGEBIETE (Int. Cl.4)**<br>C 07 C 43/295<br>C 08 G 65/00 |
| A | DE-A-1 720 830 (GENERAL ELECTRIC CO.) * Ansprüche 1, 4; Seite 5, Zeilen 19-25; Beispiel 5 * ----- | 1 | |

Der vorliegende Recherchenbericht wurde für alle Patentansprüche erstellt.

| Recherchenort | Abschlußdatum der Recherche | Prüfer |
|---|---|---|
| BERLIN | 14-11-1986 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument